(19) Europäisches Patentamt
European Patent Office
Office européen des brevets

(11) Publication number: **0 182 522 B1**

(12) **EUROPEAN PATENT SPECIFICATION**

(45) Date of publication of patent specification: **19.06.91** (51) Int. Cl.[5]: **C12P 17/18**

(21) Application number: **85307783.2**

(22) Date of filing: **28.10.85**

Divisional application 88115979 filed on 28.09.88.

The file contains technical information submitted after the application was filed and not included in this specification

(54) **Preparation of clavulanic acid and its salts and esters.**

(30) Priority: **27.10.84 ES 537157**
**27.10.84 ES 537158**
**27.10.84 ES 537159**
**27.10.84 ES 537160**

(43) Date of publication of application:
**28.05.86 Bulletin 86/22**

(45) Publication of the grant of the patent:
**19.06.91 Bulletin 91/25**

(84) Designated Contracting States:
**AT BE CH DE FR GB IT LI LU NL SE**

(56) References cited:
**GB-A- 1 508 977**

**JOURNAL OF CHEMICAL TECHNOLOGY AND BIOTECHNOLOGY, vol. 31, 1981, pages 127-134, Society of Chemical Industry, Blackwell Scientific Publications, GB; G. LILLEY et al.: "Control of the production of cephamycin C and thienamycin by streptomyces cattleya NRRL 8057"Pages 127,129,132**

(73) Proprietor: **Beecham Group p.l.c.**
**SB House Great West Road**
**Brentford Middlesex TW8 9BD(GB)**

(72) Inventor: **Puentes, José Luis Fernandez**
**c/o Antibioticos S.A. Bravo Murillo 38**
**28015 Madrid(ES)**
Inventor: **Valle, Miguel Angel Moreno**
**c/o Antibioticos S.A. Bravo Murillo 38**
**28015 Madrid(ES)**
Inventor: **Maldonado, Francisco Salto**
**c/o Antibioticos S.A. Bravo Murillo 38**
**28015 Madrid(ES)**
Inventor: **Izard, Tomas Olleros**
**c/o Antibioticos S.A. Bravo Murillo 38**
**28015 Madrid(ES)**
Inventor: **Pla, Luis Costa**
**deceased**
**28015 Madrid(ES)**
Inventor: **Sousa-Faro, José Maria Fernandez**
**c/o Antibioticos S.A. Bravo Murillo 38**
**28015 Madrid(ES)**

**EP 0 182 522 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

**CHEMICAL ABSTRACTS, vol. 98, no. 19, 9th May 1983, page 382, no. 159073w, Columbus, Ohio, US; C.H. PAN et al.: "Methyl oleate-based fermentation medium for cephalosporin C production", & DEV. IND. MICROBIOL. 1982, 23, 315-23**

**CHEMICAL ABSTRACTS, vol. 91, no. 11, 10th September 1979, page 603, no. 89456c, Columbus, Ohio, US; J.S. LEE et al.: "Effects of carbon sources and other process variables in fed-batch fermentation of penicillin", & MISAENGMUL HAKHOE CHI 1978, 16(1), 21-9**

(74) Representative: **Ablewhite, Alan James et al**
**MARKS & CLERK 57/60 Lincoln's Inn Fields**
**London WC2A 3LS(GB)**

## Description

### Technical Field

The present invention relates to the preparation of clavulanic acid, and pharmaceutically acceptable salts and esters thereof.

Clavulanic acid, (2R,5R,Z)-3-(2-hydroxyethylidene)-7-oxo-4-oxa-1-azabicyclo-[3,2,0]-heptane-2-carboxylic acid, is a known compound of structure:

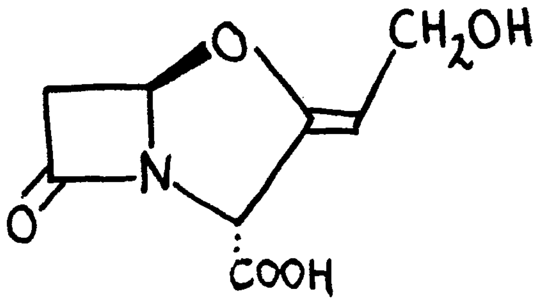

This compound, and its salts and esters, act as beta-lactamase inhibitors, and inhibit the action of beta-lactamases produced by both gram-positive and gram-negative organisms. They are therefore employed in pharmaceutical compositions to prevent inactivation of beta-lactam antibiotics. Furthermore, clavulanic acid is itself believed to have an anti-bacterial activity.

### Prior Art

Clavulanic acid is produced from various strains of micro-organism, for example the strains belonging to the genus Streptomyces such as S. clavuligerus NRRL 3585 (USA Patent 4,110,165), S. jumonjinensis NRRL 5741 (British Patent 1,563,103), S. katsurahamanus IFO 13716 (Japanese Patent 83,009,579), and Streptomyces sp. P6621 FERM 2804 (Japanese Patent 55,162,993).

Modifications in the fermentation process have been used to increase the yield of clavulanic acid. For example, British patent 1,571,888 discloses that strict pH control to a range of 6.3 to 6.7 can markedly increase the yield.

More frequently, attempts to increase yield have involved the purification process. For example, clavulanic acid salts are generally more stable than the free acid. Consequently, salts such as alkali metal and alkaline-earth metal salts have been employed to enhance the extraction yield.

USA Patent 4,110,165 describes an extraction based upon the differential solubility between aqueous and organic phases of clavulanic acid and its salts. Extraction is followed by several purifications in adsorbent material, and is thus lengthy and expensive.

The direct crystallization of clavulanic acid from the organic phase is therefore desirable. In Spanish Patent 494,431, clavulanic acid is directly crystallized from the organic phase by addition of an amine such as t-butylamine, followed by conversion to a potassium salt by exchange with a base.

The lithium salt is also considered a useful intermediate in the preparation of clavulanic acid, due to its low solubility and its easy conversion to salts or esters, acceptable for pharmaceutical formulations. For example, USA Patent 4,490,294 discloses the reaction of a solution of clavulanic acid with an aqueous ionic lithium compound, thereby to yield lithium clavulanate. British Patent 1,543,563 discloses another route to lithium clavulanate, utilizing the adsorption of filtered broth in granulated carbon, an ion-exchange resin treatment and crystallization of lithium clavulanate.

Crystallization from isopropanol of lithium clavulanate, after direct adsorption of the fermentation broth in the resin, has also been suggested. Such a process is difficult and gives poor yields.

Hence, the fermentation and purification methods of the available technology have produced impure clavulanic acid in yields which are relatively low.

### Description of the Invention.

According to a first aspect of the present invention there is provided a method for the production of clavulanic acid by batch fermentation of S. clavuligerus in a culture medium containing an assimilable

carbon source characterised in that the carbon source is added to the medium during the fermentation either continuously or intermittently.

Without being limited by theory, it is believed that the controlled addition of the carbon source leads to the absence of catabolic regulation. Regardless of theory, it is observed that a marked improvement can be obtained by taking care to prevent the carbon level from being relatively high at any time during the fermentation.

## Preferred Embodiments

For preference, the level of assimilable carbon source is kept below 1% (the percentages are on a weight/volume basis for solid carbon sources, and volume/volume for liquid carbon sources). Correspondingly, it is preferred to add the carbon source in low amounts to the production medium, either batchwise or continuously. Any addition of carbon source is preferably in an amount of not more 1%, or better still not more than 0. 5%, relative to the medium. Indeed, the carbon level in the medium is preferably maintained at below 0. 25%, such as <0. 15%.

Any assimilable carbon source can be employed, including a polyol or a carbohydrate, but more especially a chemically defined carbon source (that is, a carbon compound of chemically known structure). The defined carbon source typically has a molecular weight of below 500, and suitably comprises up to 12 carbon atoms in the molecule, such as in a mono- or di-saccharide, or a linear polyol Examples of such compounds include maltose and glycerol. In an alternative preferred form of the present invention, the assimilable carbon source is a dextrin, starch or dextrinized starch. More generally, the carbon source is usually water soluble or water miscible.

The use of S. clavuligerus NRRL 3585, is currently preferred. For such a micro-organism the preferred carbon sources include maltose, glycerol, dextrins, and optionally dextrinized starchs. Glucose, fructose, sucrose and lactose are not so readily assimilable by S. clavuligerus, and are therefore less preferred for such micro-organisms.

The carbon source can be incorporated continuously or intermittently, preferably subject to the constraint that the level of carbon source does not rise at any time above 2%. For instance, incorporation can be continuously, using constant metering of slight amounts of carbon source in to the medium. The continuous addition is suitably effected at a rate of around 0.01 to 0.1%/hour, more preferably from about 0.5 to 1.5%/day, particularly about 0.05%/hour. In an alternative mode, incorporation is effected batch-wise in accordance with a set protocol. For example, intermittent addition can be at spaced intervals ranging normally from every minute to every 6 hours. The daily addition may total 0.25% to 1.5%, although the optimum daily amount ranges from 0.5 to 1.5%%.

The incorporation need not necessarily follow a constant pattern. Thus, an incorporation of 0.5 or 1%/day can be maintained over the first 60 hours, followed by 1 or 0.5%/day, respectively, for the rest of the fermentation. Furthermore, the amount of carbon source initially present in the medium upon inoculation preferably ranges from 0% to 2%, more preferably from 0% to 0.5%, and most preferably from 0% to 0.05%.

It is preferable to add the carbon source continuously or intermittently in a sterilized manner, and to provided means to ensure and maintain this sterility. Furthermore, it is preferable that solid carbon sources are added in solution in water. Liquid carbon sources may be added directly or may be diluted appropriately.

The fermentation medium can contain other suitable culture components, including an organic nitrogen source. Such nitrogen sources can comprise oleaginous seed, optionally defatted meals, protein hydrolysates or extracts. Mineral salts (particularly chlorides, sulphates, carbonates or phosphates) and defoaming agents of mineral or organic origin can added where appropriate, together with other known medium components.

The optimum fermentation temperature is ordinarily between 24 and 30° C, particularly from 26 to 28° C, but the invention is not tied to such temperatures since fermentation can take place above or below these limits. The fermentation is typically effected on a batch system taking from 1 to many days, more usually from 24 to 240 hours, and especially 100 to 200 hours.

The most suitable fermentation vessel for the invention is a conventional aerobic fermentation vessel provided with means for agitation and aeration. The volume of such vessels can vary for instance from 1 liter and 225 m3. The working volume is preferably from 25 to 75% of the total capacity.

In working up the broth, the solids are preferably first removed from the fermentation broth by filtration or centrifugation. It is also preferred that the broth is acidified to a pH of 1 to 3, preferably around pH 2, and clavulanic acid extracted by adding a water-immiscible solvent, with the two phases being separated for

example by centrifugation, thereby giving the clavulanic acid in the water-immiscible phase.

The present invention includes as a preferred embodiment a new process for purifying clavulanic acid from solution as its lithium salt, and for optionally converting the lithium clavulanate to other salts or to esters of clavulanic acid.

Thus, more specifically, in accordance with this embodiment there is provided a method for the production of clavulanic acid as defined above, wherein the clavulanic acid is purified by:

(i) mixing the dissolved impure clavulanic acid with dissolved lithium 2-ethylhexanoate, the acid and the salt each being preferably dissolved in a water-immiscible solvent, more preferably the same solvent;

(ii) isolating lithium clavulanate; and

(iii) optionally converting the lithium salt to another salt or to an ester.

By such a process it is possible to obtain both enhanced yield and purity. The process is of general applicabilty to the purification of clavulanic acid fermentation broths, but it is especially suited to purifying extracts produced in accordance with the process of the first aspect of this invention. As mentioned above, such a fermentation can readily lead to a solution of impure clavulanic acid in a water-immiscible solvent, suited for use in the step (i).

The prefered organic solvents are ethyl acetate, methyl acetate, amyl acetate, methyl isobutyl ketone or n-butyl alcohol, with n-butyl alcohol being most preferable. Extraction of the broth is preferably conducted at a temperature in the range 4 to 10 °C, with the purpose of minimizing losses due to decomposition of the clavulanic acid.

Following extraction, crystallisation may be carried out by the addition of a solution of lithium 2-ethylhexanoate, suitably prepared in known manner. For preference, the concentration of the clavulanic acid in the organic solvent is more than 10 mg/ml. Crystallization is best performed at low temperatures, normally between 4 and 10 °C. Since lithium clavulanate crystallizes with one quarter of a molecule of water, it is recommended that crystallization is performed in the presence of a small concentration of water, such as 2 to 4% in the organic solvent. Excess lithium 2-ethylhexanoate is preferably used. Addition can take place slowly under stirring and cooling. The stirring is best maintained for a period of time after all lithium 2-ethylhexanoate has been added.

The resultant lithium clavulanate can then be further processed, and is preferably filtered through a glass plate, washed with isopropanol, acetone and ether, and dried under vacuum.

The lithium clavulanate may be recrystallized. The recrystallization can be carried out by adding ato aqueous lithium clavulanate an excess of an organic solvent in which the solubility of the lithium salt is very low, preferably isopropanol. Alternatively, and more preferably, a concentrated solution of another lithium salt is added to aqueous lithium clavulanate to cause the recrystallization. Suitable lithium salts include organic or inorganic lithium salts having high solubility in water. Lithium chloride is most preferable. Suitably, the concentration of the lithium salt solution is more than 50%, and sufficient solution is added so that the final concentration is more than 25%. Addition of the lithium salt solution preferably takes place slowly, while the lithium clavulanate solution is stirred.

The recrystallized lithium clavulanate may be further purified, for instance it can be separated by filtration, washed with acetone and dried under vacuum, thereby obtaining a yield of more than 80% and a purity above 85%.

Lithium clavulanate prepared in accordance with this invention can be converted to other salts by ion-exchange procedures, using cationic exchange resins, in the form of the desired cation, preferably sodium or potassium, and eluting with water. Furthermore, esters can be made in accordance with known procedures.

For use as an alternative to lithium salt formation, or more often in addition thereto, the present invention further includes a preferred embodiment comprising extraction of the clavulanic acid, obtained by the method of the invention, using ion-exchange resins which permit a higher exchange resin adsorption capacity. Examples of suitable resins are described, for instance, in UK Patent 1,543,563. The resin is preferably a weak basic ion exchange resin of the IRA-68 type and is preferably in the form of the acetate.

The fermentation broth suitably has an initial activity of more than 800 $\mu$g/ml of clavulanic acid, in order to obtain a good crystallization of the lithium clavulanate, after adsorption of the broth in the ion exchange resin, elution thereof with a lithium salt and subsequent crystallization for example from isopropanol.

Before adsorption of the broth in the resin, it is best to effect a clarification.

In accordance with a further preferred mode of operating the present invention, the fermentation broth obtained according to the invention is treated with an aggregating agent to aggregate the mycelium.

Aggregation of the mycelium permits a better filtration. A suitable aggregating agent is the filter coadjuvant Praestol (trademark of Bayer AG, Germany). The coadjuvant is preferably added to the broth at a concentration of from 0.1 to 0.5%, and the mix stirred for about 15 minutes before filtration. The filtration

itself is suitably effected by passage through a bed of inorganic filter material such as Dicalite (typically about 6% w:v) in a Buchner funnel (Dicalite is a trademark of Dicalite Ltd). If the first volumes of filtration are not well clarified, Refiltration through the same Dicalite prelayer can be employed. The broth can also be clarified, after filtration, by centrifugation (typically 30 minutes at 10,000 rpm).

Another improvement in the operation of the method of this invention is an increase in the adsorption capacity of the ion-exchange resin achieved by deproteinization of the filtered broth.

The deproteinization is preferably effected by acidification, usually to around pH 4, or by treatment with a precipitating solvent such as acetone.

In an embodiment of the invention, the pH of the filtered broth is adjusted to about 4.0 with mineral acid, such as hydrochloric or sulphuric acid. Once the pH has been adjusted, the supernatant is adsorbed in the resin. The broth is preferably first allowed to stand, and any turbidity eliminated by centrifugation. To prevent product losses due to the pH of around 4, the mixture can be brought back to about pH 6 before adsorption on the resin. The adsorption capacity of the resin in the form of acetate at a pH of 6 is increased in this manner. Preferably the ratio of filtered broth:resin (v/v) is not more than 10:1

The filtered broth can also be deproteinized by treatment with a precipitating solvent, such as acetone. In an embodiment of the present invention, one volume of broth is mixed with about one volume of acetone. For preference, the broth is stirred for 5 to 10 minutes with acetone, allowed to stand for 20 minutes, and the precipitate separated by centrifugation or filtration. The broth thus clarified is suitable for its adsorption in the resin. The acetone can be recoverable by distillation under reduced pressure before adsorption of the broth. Whether the acetone has been removed or not, the filtered broth can be adsorbed in the resin at a ratio of from 10:1 to 12:1 (initial volume of filtered broth:volume of resin) without considerable losses.

Crystallization of lithium clavulanate from the eluates of ion exchange resins has previously led to formation of gums and in some cases a low purity. To minimize this problem, it is preferred to wash or pre-elute the resin with 0.5% sodium chloride and then with water, before elution with the lithium salt, such as 5% lithium chloride.

Crystallization of the lithium clavulanate can be further improved by treatment of the eluate with acetone. The eluate of the resin is stirred for example with two volumes of acetone and allowed to stand for 15 minutes. The precipitate is separated by centrifugation to allow crystallization of the lithium clavulanate from isopropanol, after the acetone has been eliminated.

The present invention is illustrated in non-limiting manner by the following examples.

EXAMPLE 1

The following medium was prepared:

| | | |
|---|---|---|
| fishmeal | 2.0 | g |
| glycerol | 1.5 | g |
| soluble starch | 1.5 | g |
| calcium carbonate | 0.2 | g |
| distilled water to | 100 | ml |

The pH was adjusted to 7, and 40 ml aliquots were placed in 250 ml Erlenmeyer flasks and sterilized.

The medium was then seeded with a suspension of spores of S. clavuligerus NRRL 3585 and incubated for 2 days at 28° C under agitation. The culture was employed to seed, to 2%, 500ml of the same medium in a 2-liter Erlenmeyer flack. Culturing was also carried out on a rotary shaker at 28° C for 2 days.

500 ml of the culture thus prepared were used to seed a 340-liter stainless steel vessel fitted for agitation, aeration and temperature control. The vessel contained 100 liters of a sterilized medium of the following composition:

| | % |
|---|---|
| soybean meal | 3 |
| corn dextrin | 3 |
| soybean oil | 0.5 |
| $KH_2PO_4$ | 0.1 |
| defoamer (ucon) | 0.005 |
| tap water to final volume | |

The seeded medium was cultured at 28°C, receiving air with stirring for 45 hours. Thereafter, a further 340-liter vessel was seeded, to 7%, with 150 liters of the same medium. Incubation took place at 28°C for 30 hours, under stirring and aeration.

Upon termination, this culture was used to seed, to 7%, a clavulanic acid production medium contained in a tank. The tank had a total capacity of 800 liters, and 425 liters of the following medium were added:

| | | |
|---|---|---|
| soybean meal | 1.25 | % |
| peanut meal | 1.25 | % |
| distiller's dried grain | 0.5 | % |
| $KH_2PO_4$ | 0.1 | % |
| defoamer (ucon) | 0.005 | % |
| tap water to final volume | | |

Sterilization took place at 121°C for 20 minutes, after adjusting the pH to 6.7. Glycerol was added intermittently every hour as follows:

| time (h) | rate (ml/hr) | total glycerol |
|---|---|---|
| 0 to 12 | 180 | 0.5% |
| 13 to 96 | 190 | 3.7% |
| 97 to 160 | 100 | 1.5% |

Fermentation was conducted at 26°C under stirring and aeration. After 160hrs the level of clavulanic acid was high, at 1403 μg/ml.

EXAMPLES 2 AND 3

COMPARATIVE EXAMPLES 1 AND 2

The effect of adding the gylycerol gradually during fermentation was compared directly with the effect of including all the glycerol at the start of the fermentation.

The following table illustrates the results:

| example* | % glycerol | | clavulanic acid | |
|---|---|---|---|---|
| | initial | Addition | 112 h | 160 h |
| E2 | 0 | 5.7 | - | 1403 |
| E3 | 1 | 4.8 | 402 | 669 |
| CE1 | 3.5 | 0 | 359 | 273 |
| CE2 | 5.5 | 0 | 198 | 199 |

* E, Example; CE, Comparative Example

A substantial improvement in production is noted when the assimilable carbon source is incorporated during the fermentation.

EXAMPLE 4

40 liters of the same fermentation production medium as in Example 1 were seeded with the same inoculum in a 75 litre fermenter. A sterile, aqueous solution of 50% maltose was then added continuously using a continous addition diaphragm pump, at the rate of 800 ml daily (1% maltose/day) for 112 hours, at which time the broth was collected For extraction. The Fermenter was agitated and aerated in conventional fashion. The concentration of the clavulanic acid observed in the broth after 112 hours was 1,424 $\mu$g/ml.

EXAMPLE 5

7.5 Liters of n-butanol were added to 7.5 liters of filtered broth from Example 1, and the pH was adjusted to 2 with cold 4N sulphuric acid. The mixture was centrifuged to 16,000 g, thereby separating the two phases, aqueous and butanolic. The butanol phase was concentrated under reduced presure at 35° C to a volume of 200 ml.

In this way, a solution of clavulanic acid in 200 ml of n-butanol was obtained with a concentration of 26.7 mg/ml. 5 ml of water were added, giving a moisture level of 2.52%. To this solution, under stirring, were added 100 ml of 10% solution of lithium 2-ethylhexanoate in n-butanol. The total volume was concentrated to 150 ml, it was stirred for 2 hours at 5° C and filtered through a filtering plate. The crystalline form was washed with 100 ml of isopropanol, 100 ml of acetone and 100 ml of ether, followed by drying at 40° C under vacuum, to give 7.57 g of the product, purity 54%, moisture 3.2% and 23.5% ash (as lithium sulphate).

EXAMPLE 6

10 g of lithium clavulanate obtained as in Example 5 were dissolved in 50 ml of water, adding thereto 75 ml of a solution of 50 w/v of lithium chloride slowly under stirring. Crystallization started, and the mix was stirred for half an hour at 5° C and filtered through a glass flask, washed with acetone and dried under vacuum at 40° C, obtaining 5.27 g of product, yield 84% amd purity 80%.

Physical characteristics of the recrystallized lithium clavulanate

IR ($cm^{-1}$, Nujol (trade mark)): 3420, 3010, 1765, 1680, 1620, 1400, 1340, 1325, 1300, 1220, 1200, 1130, 1100, 1060, 1050, 1020, 990, 970, 950, 900, 880, 850, 730, 708.

$^{20}[\alpha]_D$ = 451 (c = 145 $\mu$g/ml in water)

UV $\lambda_{max}$ = 259 (c = 10 $\mu$g/ml in O.IN NaOH)

(extinction molar coefficient, ϵ = 19,451)
lithium content (by absorption) = 3.3%
ash = 26.5%, as lithium sulphate.

EXAMPLE 7

8000 ml of a total fermentation broth (activity 1280 μg/ml of clavulanic acid) prepared in accordance with Example 1 or 4, were stirred for 15 minutes with filtration coadjuvant, such as 0.5% Praestol. The broth was mixed with Dicalite at a ratio of 6:1 (w/v) and filtered carefully through filtering paper in a Buchner flask. After the broth had been filtered and clarified, the pH was adjusted to 4.0 for deproteinization. The precipitate was separated by centrifugation. The supernatant was adsorbed at pH 6.0 in a basic IRA-68 (Trade Mark) resin, in a column having a v/v Filtered broth/resin ratio of 10:1. This resin was in the form of the acetate, pH 4.0.

Once the broth was adsorbed, the resin was washed with 0.5% sodium chloride and then with water. The clavulanic acid was eluted as the lithium salt using lithium chloride. The eluate of the resin (510 ml, activity: 4,500 μg/ml of clavulanic acid) was treated with two volumes of acetone, and the precipitate separated by centrifugation. The supernatant was mixed with 5 volumes of isopropanol, and again the precipitate separated by centrifugation. The supernatant was concentrated, allowing the lithium salt to crystallize at 4° C for 24 hours.

1182 mg were obtained, yield 11.5% and purity 96.92%/

EXAMPLE 8

A total fermentation broth (5,000 ml, activity: 1396 μg/ml of clavulanic acid) was filtered as described in Example 7.

The filtrate was mixed with one volume of acetone under continous stirring, allowing it to settle for 15 to 20 minutes. The sediment was removed by centrifugation and the acetone was taken off on a rotary evaporator.

The clarified broth was adsorbed in an IRA-68 ion-exchange resin as described in Example 7 (broth: resin ratio of 10:1 v/v). In this same manner the resin was washed and the lithium clavulanate was eluted to be crystallized from isopropanol. The yield was 20%. The eluate was separated into two fractions. The first fraction gave 650 mg, purity 69.3% and total yield 9%. The second fraction of the eluate gave 770 mg, purity 98% and total yield 11%.

The 69.3% pure crystallized lithium clavulanate contained 5% lithium and 35.4% ash as lithium sulphate. The lithium clavulanate was recrystallized from 55% lithium chloride, to give a product with the following characteristics:
ϵ = 15,908 (in 0.1 N NaOH)
lithium content = 3.6%
ash = 25%, as lithium sulphate
purity = 95%
IR (Nujol, $cm^{-1}$) 3430, 3010, 1760, 1680, 1615,
1375, 1300, 1210, 1130, 1105, 1065, 1050, 1030,
995, 980 ,950, 900, 885, 855, 740, 710.

EXAMPLE 10

A total broth (4,000 ml with 300 λ/ml of clavulanic acid) was treated with Praestol and filtered as in Example 7. The broth was clarified at pH 6.0 and passed over a mixed column of IRA-68 and XAD-2 (Trade Mark) resin mixed at a ratio of 40:1 (volume of filtered broth: volume of resin).

The eluate of the pre-column containing practically 100% of the initial clavulanic acid activity was adsorbed in a column at a broth: resin ratio (v/v) of 10:1. The resin was washed with 0.5% sodium chloride and then with water. The lithium clavulanate was eluted with lithium chloride. Once the clavulanate was crystallized, as described in Example 7, 181 mg of the first product were obtained, purity 95.3% and yield 15.08% The physical properties were similar to those of the product of Example 7.

EXAMPLES 11 to 16

Different treatments were tried as part of adsorption of the broth in IRA-68 resin, acetate form. For

Example 11, the broth was filtered and subjected to resin treatment was adopted. For Example 12, the resin was first washed with sodium chloride solution. For Example 13, the broth was filtered with coadjuvant. For Example 14, deproteinization of the broth at pH 4 was effected in addition to the procedure of Example 13. For Example 15, deproteinization of the broth with acetone was effected in addition to the procedure of Example 13. For Example 16, post treatment of the eluate with acetone was effected in addition to the procedure of Example 14.

| example | exhaustion | wash | eluate | yield | purity |
|---|---|---|---|---|---|
| 11 | 30-40 | 0-5 | 10-15 | 5 | variable |
| 12 | 30 | 5-10 | 10-15 | 5 | 50-80 |
| 13 | 0-7 | 0-1 | 50 | 5-10 | 60-80 |
| 14 | 0-2 | 0 | 60-70 | 10-20 | 80 |
| 15 | 0-2 | 0 | 70 | 20-22 | 80-90 |
| 16 | 0-2 | 0 | 70 | 20-25 | 90-95 |

The yields refer to the first collection of lithium clavulanate crystals.

The optimum process involves a filtration in the presence of a coadjuvant, a deproteinization at pH 4.0 and treatment with acetone prior to crystallization.

EXAMPLES 17 AND 18

The process of the preceding Examples can be further improved by first passing the filtered broth over a pre-column of IRA-68 or IRA-68 mixed with XAS-2 (Trade Mark), then deproteinizing or not at pH 4.0 or with acetone and the direct adsorption in the ion-exchange resin, as already indicated. For Example 17, a pre-column treatment was included in the procedure of Example 13, while For Example 17 a deproteinization at pH 4 and post-treatment with acetone was included in the procedure of Example 18.

| example | exhaustion | wash | eluate | yield | purity |
|---|---|---|---|---|---|
| 13 | 0-7 | 0-1 | 50 | 5-10 | 60-80 |
| 17 | 5 | 0 | 40-50 | 10-20 | 80 |
| 18 | 0-2 | 0 | 50 | 20-30 | 90-95 |

An increase in the adsorption capacity of the resin and better yield and purity of the end product was obtained in the Examples 17 and 18.

## Claims

1. A method for the production of clavulanic acid by the batch fermentation of S. clavuligerus in a culture medium containing an assimilable carbon source characterised in that the carbon source is added to the medium during the course of the fermentation, either continuously or intermittently.

2. A method according to claim 1, wherein the level of assimilable carbon source is kept below 0. 5% (w/v).

3. A method according to claim 2, wherein the carbon level is maintained at below 0.25% (w/v) during the productive fermentation.

4. A method according to any of claims 1 to 3, wherein the carbon source is incorporated continuously or intermittently at a rate of from 0. 5 to 1. 5%/day.

5. A method according to claim 4, wherein the carbon source is incorporated at a rate of about 0.05%/hour.

6. A method according to any preceeding claim, wherein the clavulanic acid is purified from solution using the steps of
   (i) mixing the dissolved impure clavulanic acid with dissolved lithium 2-ethylhexanoate;
   (ii) isolating lithium clavulanate; and
   (iii) optionally converting the lithium salt to another salt or to an ester.

7. A method according to any preceding claim, wherein the clavulanic acid is purified using an ion-exchange resin.

8. A method according to any preceding claim, wherein the fermentation broth is treated with an aggregating agent to aggregate the mycelium.

9. A method according to any preceding claim, wherein the fermentation broth is filtered and deproteinized.

10. A method for the production of clavulanic acid, the method involving the steps of
   (a) providing a culture medium;
   (b) inoculating the culture medium with S. clavuligerus micro-organism;
   (c) initiating batch fermentation of the culture medium by the micro-organism in the presence of an assimilable carbon source;
   (d) obtaining a fermented broth containing clavulanic acid, and
   (e) purifying the clavulanic acid from the broth;
   characterised in that carbon source is added to the medium during the course of the fermentation, either continuously or intermittently.

**Revendications**

1. Procédé pour la production d'acide clavulanique par la fermentation discontinue de S. clavuligerus dans un milieu de culture contenant une source de carbone assimilable, caractérisé en ce que la source de carbone est ajoutée au milieu au cours de la fermentation, soit en continu soit par intermittence.

2. Procédé suivant la revendication 1, caractérisé en ce que la concentration de la source de carbone assimilable est maintenue en dessous de 0,5 % en p/v.

3. Procédé suivant la revendication 2, caractérisé en ce que la concentration de carbone est maintenue en dessous de 0,25 % en p/v pendant la fermentation productive.

4. Procédé suivant l'une quelconque des revendications 1 à 3, caractérisé en ce que la source de carbone est incorporée en continu ou par intermittence à raison de 0,5 à 1,5 % par jour.

5. Procédé suivant la revendication 4, caractérisé en ce que la source de carbone est incorporée à raison d'environ 0,05 % par heure.

6. Procédé suivant l'une quelconque des revendications précédentes, caractérisé en ce que l'acide clavulanique est purifié à partir d'une solution au moyen des étapes suivantes :
   (i) mélange de l'acide clavulanique impure dissoute avec du 2-éthylhexanoate de lithium dissous,
   (ii) isolation du clavulanate de lithium, et
   (iii) conversion éventuelle du sel de lithium en un autre sel ou en un ester.

7. Procédé suivant l'une quelconque des revendications précédentes, caractérisé en ce que l'acide clavulanique est purifié au moyen d'une résine d'échange d'ion.

8. Procédé suivant l'une quelconque des revendications précédentes, caractérisé en ce que le bouillon de fermentation est traité avec un agent d'agrégation pour agglomérer le mycélium.

9. Procédé suivant l'une quelconque des revendications précédentes, caractérisé en ce que le bouillon de fermentation est filtré et déprotéinisé.

10. Procédé pour la production d'acide clavulanique impliquant les étapes suivantes :
(a) fourniture d'un milieu de culture ;
(b) inoculation du milieu de culture avec un microorganisme S. clavuligerus ;
(c) initiation d'une fermentation discontinue du milieu de culture par le microorganisme en présence d'une source de carbone assimilable ;
(d) obtention d'un bouillon fermenté contenant l'acide clavulanique ; et
(e) purification de l'acide clavulanique à partir du bouillon ;
caractérisé en ce que la source de carbone est ajoutée au milieu au cours de la fermentation, soit en continu soit par intermittence.

## Ansprüche

1. Verfahren zur Herstellung von Clavulansäure durch Chargen-Fermentation von S. clavuligerus in einem eine assimilierbare Kohlenstoffquelle enthaltenden Kulturmedium, **dadurch gekennzeichnet**, daß die Kohlenstoffquelle dem Medium während des Verlaufs der Fermentation entweder kontinuierlich oder absatzweise zugegeben wird.

2. Verfahren nach Anspruch 1, wobei die Menge der assimilierbaren Kohlenstoffquelle unter 0,5 % (Gew./Vol.) gehalten wird.

3. Verfahren nach Anspruch 2, wobei die Kohlenstoffmenge unter 0,25 % (Gew./Vol.) während der produktiven Fermentation gehalten wird.

4. Verfahren nach einem der Ansprüche 1 bis 3, wobei die Kohlenstoffquelle kontinuierlich oder absatzweise in einer Menge von 0,5 bis 1,5 %/Tag zugegeben wird.

5. Verfahren nach Anspruch 4, wobei die Kohlenstoffquelle in einer Menge von etwa 0,05 %/Tag zugegeben wird.

6. Verfahren nach einem vorangehenden Anspruch, wobei die Clavulansäure aus der Lösung unter Anwendung der Stufen
(i) Mischen der gelösten unreinen Clavulansäure mit gelöstem Lithium-2-ethylhexanoat;
(ii) Isolierung von Lithium-Clavulanat; und
(iii) gegebenenfalls Umwandlung des Lithiumsalzes in ein anderes Salz oder in einen Ester,
gereinigt wird.

7. Verfahren nach einem vorangehenden Anspruch, wobei die Clavulansäure unter Verwendung eines Ionenaustauscherharzes gereinigt wird.

8. Verfahren nach einem vorangehenden Anspruch, wobei die Fermentationsbrühe mit einem Aggregationsmittel zur Aggregation des Mycels behandelt wird.

9. Verfahren nach einem vorangehenden Anspruch; wobei die Fermentationsbrühe filtriert und deproteiniert wird.

10. Verfahren zur Herstellung von Glavulansäure, wobei das Verfahren die Stufen
(a) Bereitstellung eines Kulturmediums;
(b) Überimpfung des Kulturmediums mit S. clavuligerus-Mikroorganismen;
(c) Start einer Chargen-Fermentation des Kulturmediums durch den Mikroorganismus in Gegenwart

einer assimilierbaren Kohlenstoffquelle;

(d) Erhalt einer Clavulansäure enthaltenden fermentierten Brühe, und

(e) Reinigung der Clavulansäure aus dem Bad

umfaßt, **dadurch gekennzeichnet**, daß die Kohlenstoffquelle dem Medium im Verlauf der Fermentation entweder kontinuierlich oder absatzweise zugegeben wird.